# EUROPÄISCHE PATENTANMELDUNG

(11) **EP 2 002 792 A2**
(43) Veröffentlichungstag der Anmeldung: **17.12.2008**
(21) Anmeldenummer: 08010744.4
(22) Anmeldetag: 12.06.2008
(51) Int. Cl.: A61B 17/16, B25B 23/14

(54) **Montierinstrument für einen Marknagel**

(30) Priorität: 12.06.2007 DE 202007008234 U
(71) Anmelder: Englert, Thomas, 63762 Grossostheim (DE)
(72) Erfinder: Englert, Thomas, 63762 Grossostheim (DE)

(57) **Zusammenfassung**

Montierinstrument (W) für einen Marknagel (N) mit zumindest einem durch eine relative Drehbewegung im Markkanal aufweitbaren Spiralfederkörper (17,22), mit einem am Montierinstrument (W) vorgesehenen Haltegriff (1) für ein Positionierelement (4) und zumindest einer Drehhandhabe (5,10), die mit einem relativ zum Positionierelement drehbaren Spiralfederkörper-Drehelementrohr (12,7) in Verbindung steht, in der Verbindung zwischen der Drehhandhabe (5,10) und dem Spiralkörper-Drehelementrohr (12,7) eine Drehrastvorrichtung (R) angeordnet ist, die während einer Drehung unter einem von der Drehhandhabe auf das Spiralfederkörper-Drehelementrohr aufgebrachten Drehmoment schlagartig variierende Drehkraftimpulse (42) generiert.

## Beschreibung

Die Erfindung betrifft ein Montierinstrument gemäß Oberbegriff des Anspruchs 1.

Es sind zum Einsetzen von Marknägeln in den Markkanal eines Knochens Montierinstrumente bekannt, die untereinander den Haltegriff für das Positionierelement, eine erste Drehhandhabe und eine zweite Drehhandhabe aufweisen. Mit dem Haltegriff wird ein Kernrohr des Marknagels drehfest gehalten, zunächst mit der einen Drehhandhabe ein äußerer Spiralfederkörper des Marknagels bis zur Anlage an der Wand des Markkanals aufgeweitet und in einen Klemmeingriff mit dem Knochen gebracht wird, ehe mit der anderen Drehhandhabe ein innenliegender Spiralfederkörper aufgeweitet wird, um den äußeren Spiralfederkörper zu fixieren. Das jeweils auf den Spiralfederkörper aufgebrachte Drehmoment wird mit der Erfahrung des Operateurs bestimmt. Unter ungünstigen Bedingungen wird speziell das Drehmoment für den äußeren Spiralfederkörper leicht zu hoch oder zu niedrig gewählt. Bei zu hohem Drehmoment kann der Knochen sofort oder nach Abschluss der Operation splittern. Bei zu niedrigem Drehmoment besteht die Gefahr, dass sich der Marknagel nach der Operation wieder lockert. Ein erheblicher Nachteil der bekannten Montierinstrumente besteht jedoch darin, dass das jeweils vom Operateur aufgebrachte Drehmoment gleichmäßig bis zu der vom Operateur gewählten Drehmomentgrenze gesteigert wird. Speziell der äußere Spiralfederkörper des Marknagels erstreckt sich jedoch über eine Länge von etwa 25 cm. Der Markkanal ist vor dem Einsetzen des Marknagels zwar aufgebohrt, hat dennoch naturbedingt eine über die Länge variierende Innenweite bzw. Bereiche unterschiedlicher Knochendichten. Ferner kann beim Aufweiten des äußeren Spiralfederkörpers Knochenmaterial oder dgl. abgelöst werden, das sich zwischen den Windungen des Spiralfederkörpers festsetzt, während der Spiralfederkörper aufgeweitet wird. Da das Drehmoment auf das oberste Ende des Spiralfederkörpers aufgebracht wird, und die Windungen des Spiralfederkörpers über dessen Länge variierende Aufweit- und Drehwiderstände antreffen, muss der Operateur bei Erreichen der von ihm gewählten Drehmomentgrenze davon ausgehen, dass der Spiralfederkörper über seine ganze Länge ordnungsgemäß aufgeweitet ist bzw. sich das Drehmoment über die gesamte Länge des Spiralfederkörpers gleichmäßig verteilt hat. In der Praxis lässt sich dies jedoch nicht verwirklichen, da von außen keine Einflussnahme auf tiefer im Markkanal liegende Bereiche des Spiralfederkörpers möglich sind. Es zeigt sich häufig, dass der Spiralfederkörper nur bis zu einer Tiefe im Markkanal ordnungsgemäß aufgeweitet ist, die von dem Vorliegen der Bereiche unterschiedlicher Aufweit- und Reibungswiderstände abhängt, und tieferliegende Abschnitte des Spiralfederkörpers nicht ordnungsgemäß oder gar nicht aufgeweitet sind. Dies führt zur akuten Gefahr eines späteren Lockerns des Marknagels.

Der Erfindung liegt die Aufgabe zugrunde, ein Montierinstrument der eingangs genannten Art dahingehend zu verbessern, dass die Wahrscheinlichkeit, den Spiralfederkörper ordnungsgemäß über seine ganze Länge aufzuweiten und den Marknagel über die gesamte Länge ordnungsgemäß im Markkanal zu fixieren, wesentlich erhöht werden kann. Teil der Aufgabe ist es ferner, den Operateur der Aufgabe des Wählens des Drehmoments zu entheben und das Drehmoment bis zu einer Grenze aufzubringen, bei der ein optimaler Sitz des eingesetzten Marknagels gewährleistet werden kann.

Die gestellte Aufgabe wird mit den Merkmalen des Patentanspruchs 1 gelöst.

Die Drehrastvorrichtung erzeugt unter dem aufgebrachten Drehmoment schlagartig variierende Drehkraftimpulse, die auf das obere Ende des Spiralfederkörpers einwirken. Diese Drehkraftimpulse haben die Tendenz, sich bis zum unteren Ende des Spiralfederkörpers fortzupflanzen, d.h., auch durch die Bereiche im Markkanal durchzugehen, in denen lokal ein höherer Reibungswiderstand oder Aufweitwiderstand vorliegt. Diese wie Schläge wirkenden Drehkraftimpulse nutzen das Federverhalten des Spiralfederkörpers so, dass sich die Windungen bis zum innenliegenden Ende des Spiralfederkörpers an solchen lokalen Bereichen höheren Widerstands vorbeibewegen. Die Wirkung ist ähnlich wie bei einem Schlagbohrer in Beton, der gegenüber einem normal angetriebenen Bohrer leichter eindringt und das Material leichter verdrängt bzw. abschert. So ermöglicht es das Montierinstrument, Marknägel bestimmungsgemäß korrekt zu setzen und die Gefahr eines späteren Lockerns des Marknagels im Markkanal weitestgehend zu eliminieren.

Bei einer zweckmäßigen Ausführungsform ist die Drehrastvorrichtung ein Drehmomentbegrenzer, der, vorzugsweise, sogar einstellbar ist, um den Spiralfederkörper so aufzuweiten, dass ein optimaler Sitz des Marknagels erreicht wird, ohne Gefahr, den Spiralfederkörper zu wenig oder zu stark aufzuweiten. Der Drehmomentbegrenzer kann zusätzlich zur Drehrastvorrichtung vorgesehen sein, lässt sich jedoch auf baulich einfache Weise bereits mittels der Drehrastvorrichtung realisieren, und enthebt den Operateur der Aufgabe, das Drehmoment nach Gefühl oder Erfahrung und damit oftmals als einen Kompromiss zu wählen. Die Hersteller von Marknägeln und von solchen Montierinstrumenten können nämlich anhand vieler vorab durchgeführter Versuche optimale Drehmomentwerte gegebenenfalls besser als Operateure festlegen.

In einer zweckmäßigen Ausführungsform ist die Drehrastvorrichtung ein federbelastetes Ratschensystem. Dieses Ratschensystem kann wie in Schraubwerkzeugen üblich ausgebildet sein, d.h. mit Rastkugeln oder Rastzähnen.

Besonders zweckmäßig ist jedoch eine Drehrastvorrichtung, die unter Federkraft in einen gegenseitigen Formschluss gepresste, zueinander konkav gerundete Eingriffselemente enthält. Die konkave Krümmung bietet nämlich den Vorteil, beim stärksten bzw. tiefsten gegenseitigen Eingriff aufgrund der dann am steilsten stehenden Tangenten in den Berührungsstellen die höchste Drehkraft zu übertragen, und diese Drehkraft unter Verringern der Eingriffstiefe relativ gleichförmig abnehmen zu lassen, bis schließlich beim Übereinandergleiten zweier im Eingriff befindlicher Eingriffselemente die Tangenten im Berührungsbereich annähernd parallel zur Drehrichtung sind und auch die übertragene Kraft ein Minimum ist. Es wird hier speziell auf DE-202 005 005 513 U1 hingewiesen, in der eine hier zweckmäßige Drehrastvorrichtung offenbart ist. Der Gesamtinhalt dieses Dokuments wird hiermit durch Rückbeziehung integriert.

In einer zweckmäßigen Ausführungsform weisen die Eingriffselemente kugelige, zylindrische oder konische Eingriffsflächen auf, die miteinander in Punkt- oder Linienkontakt stehen. Die Eingriffsflächen können gleich oder verschieden sein, wesentlich ist nur, dass sie konkav gekrümmt sind bzw. Kreisbögen als Erzeugende aufweisen.

Eine spezielle Ausführungsform des Montierinstruments für Marknägel mit zwei ineinandergesetzten, gegensinnigen Spiratfederkörpern und Spiralfederkörper-Drehelementen weist zwei getrennte Drehhandhaben auf. Zwischen Jeder Drehhandhabe und dem Spiralfederkörper-Drehelement ist eine sölche Drehrastvorrichtung angeordnet, die vorzugsweise ein Drehmomentbegrenzer ist. Da der innere Spiralfederkörper ohnedies nur zum Fixieren des äußeren, aufgeweiteten Spiralfederkörpers dient, könnte es ausreichen, eine Drehrastvorrichtung bzw. einen Drehmomentbegrenzer nur der Drehhandhabe für den äußeren Spiralfederkörper zuzuordnen. Gegebenenfalls wird nur eine einzige Drehhandhabe vorgesehen, die in entgegengesetzten Drehrichtungen wahlweise den einen und dann den anderen Spiralfederkörper antreibt.

Die Drehmomentbegrenzer, falls zwei vorgesehen sind, könnten auf individuelle Drehmomentgrenzen eingestellt bzw. einstellbar sein, da gegebenenfalls das Drehmoment für den inneren Spiralfederkörper niedriger sein kann als das Drehmoment für den äußeren Spiralfederkörper.

Die Rastvorrichtung kann ein an der Drehhandhabe angebrachtes Gehäuse aufweisen, in weichem die Eingriffselemente und wenigstens eine Feder untergebracht sind. Dies ergibt eine kompakte Bauweise des Montierinstrumentes.

Um die Drehhandhaben relativ zueinander leicht drehen zu können, ist es zweckmäßig, wenn zwischen dem einen Gehäuse und der anderen Drehhandhabe eine Distanzhülse, vorzugsweise aus einem gleitfähigen Kunststoff, wie Polytetrafluorethylen, angeordnet ist.

Da das Montierinstrument, wie medizinische Geräte allgemein, intensiv gereinigt und sterilisiert werden müssen, ist es zweckmäßig, wenn das Gehäuse Reinigungsöffnungen besitzt.

Um die jeweilige Drehhandhabe ordnungsgemäß auch dann drehen zu können, wenn Feuchtigkeit, Blut oder dgl. an der Drehhandhabe haften sollte, ist es zweckmäßig, randseitige Greifhilfen vorzusehen.

Bei einer alternativen Ausführungsform kann das Gehäuse gleichzeitig als die Drehhandhabe ausgebildet sein, so dass die Teileanzahl bei der Erstellung des Montierinstrumentes reduziert ist.

Anhand der Zeichnungen wird eine Ausführungsform des Erfindungsgegenstandes erläutert. Es zeigen:

**Fig. 1** eine Seitenansicht eines Montierinstrumentes für Marknägel,

**Fig. 2** eine teilweise im Schnitt ausgebildete Seitenansicht eines Marknagels,

**Fig. 3** eine vergrößerte Seitenansicht des unteren Endes des Montierinstrumentes von **Fig. 1****,**

**Fig. 4** eine Schemadarstellung einer im Montierinstrument enthaltenen Drehrastvorrichtung, und

**Fig. 5** ein Diagramm der über die Drehrastvorrichtung aufgebrachten Drehkraft über dem Drehhub.

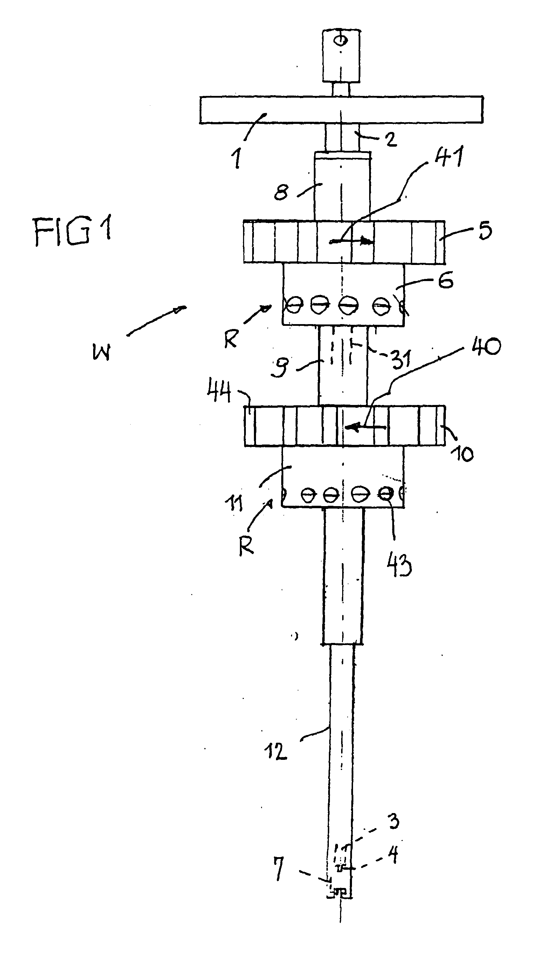

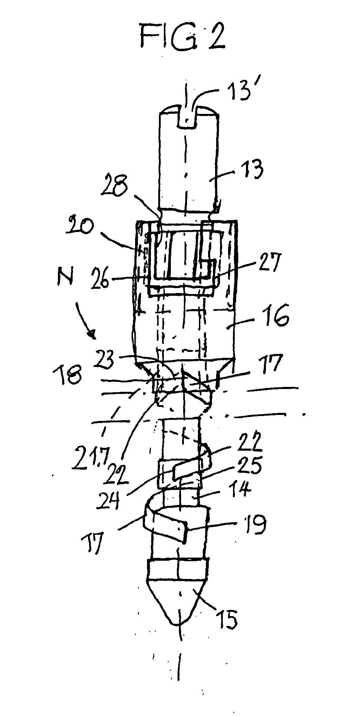

Ein in **Fig. 1** gezeigtes Montierinstrument W für Marknägel (mit zwei ineinandergesetzten Spiralfederkörpern gemäß **Fig. 2****)** weist einen Haltegriff 1 (z.B. einen Knebel) auf, der mit einer sich nach unten erstreckenden Welle oder einem Rohr 3 verbunden ist. Am unteren Ende ist ein Vorsprung 4 angeformt. Das Rohr 3 oder die Welle ist mit einem Rohr 2 am Haltegriff verbunden. Eine Distanzhülse 8 separiert den Haltegriff 1 von einer oberen Drehhandhabe 5, an deren Unterseite ein topfförmiges Gehäuse 6 angebracht ist, das eine Drehrastvorrichtung R enthält. Ausgangsseitig der Drehrastvorrichtung R ist ein Rohr 31 angebracht, das nach unten zu einem Spiralfederkörper-Drehelementrohr 7 führt, das relativ zu dem durch den Fortsatz 4 gebildeten Positionierelement drehbar ist. Zwischen dem Gehäuse 6 und einer weiteren Drehhandhabe 10 ist eine Distanzhülse 9, vorzugsweise aus Kunststoff, vorgesehen. An der weiteren Drehhandhabe 10 ist unterseitig ein Gehäuse 11 einer weiteren Drehrastvorrichtung R angesetzt, die ausgangsseitig mit einem Spiralfederkörper-Drehelementrohr 12 verbunden ist, das relativ zum Vorsprung 4 und zum Spiralfederkörper-Drehelementrohr 7 drehbar ist. In jedem Gehäuse 6, 11 sind Reinigungsöffnungen 43 ausgebildet. Die Drehhandhaben 5, 10 können randseitige Drehhilfen 44 besitzen.

Bei einer nicht gezeigten, alternativen Ausführungsform können die Gehäuse 6, 11 gleichzeitig die Drehhandhaben **5, 10** bilden. Ferner könnte bei einer alternativen Ausführungsform die Drehrastvorrichtung R der Drehhandhabe 5 für den inneren Spiralfederkörper des Marknagels entfallen. Bei einer weiteren Alternative für Marknägel mit nur einem Spiralfederkörper könnte nur eine Drehhandhabe mit einer Drehrastvorrichtung vorgesehen sein. Zweckmäßig bilden die in **Fig. 1** gezeigten Drehrastvorrichtungen R gleichzeitig Drehmomentbegrenzer, die auf eine feste Drehmomentgrenze eingestellt oder einstellbar sind. Die Drehmomentgrenzen können für beide Drehrastvorrichtungen R individuell sein.

**Fig. 2** zeigt schematisch einen Marknagel N mit einem äußeren Spiralfederkörper 17 und einem inneren, gegensinnigen Spiralfederkörper 22. Der Marknagel N hat beispielsweise eine Länge von etwa 25 bis 30 cm und in entspanntem Zustand einen Außendurchmesser von etwa 7 bis 10 mm. Der Marknagel N wird in den aufgebohrten Markkanal eines Knochens eingeführt und darin durch Aufweiten des äußeren Spiralfederkörpers **17** über die gesamte Länge fixiert, wobei der aufgeweitete Zustand des äußeren Spiralfederkörpers 17 dann durch den ebenfalls aufgeweiteten inneren Spiralfederkörper 22 fixiert wird.

Der Marknagel N besitzt einen Kopf 13 mit einer Quernut 13' zum Eingriff des Vorsprungs 4. Mit dem Kopf 13 ist eine Welle oder ein Rohr 14 verbunden, das an einem angespitzten Ende 15 befestigt ist. Unterhalb des Kopfes 13 ist eine äußere Hülse 16 drehbar gelagert, an deren unterem Ende das obere Ende des äußeren Spiralfederkörpers 17 beispielsweise bei 18 befestigt, z.B. angelötet, ist. Das untere Ende des äußeren Spiralfederkörpers 17 ist am angespitzten Ende 15 bei 19 festgelegt. Dazwischen windet sich der äußere Spiralfederkörper 17 beispielsweise ca. 30 mal, wobei die Windungsbreite (nicht gezeigt) nach unten allmählich abnehmen kann.

In der äußeren Hülse 16 ist eine innere Hülse 20 drehbar angeordnet, an deren unterem, aus der äußeren Hülse 16 vorstehenden Ende 21 bei 23 der innere Spiralfederkörper 22 festgelegt ist, dessen Windungen gegensinnig zu den Windungen des äußeren Spiralfederkörpers 22 verlaufen. Das untere Ende des inneren Spiralfederkörpers 22 ist beispielsweise bei 24 an einer auf dem Rohr oder der Welle 14 angebrachten Hülse 25 befestigt.

Die äußere Hülse 16 und die innere Hülse 20 weisen jeweils ein Spiralfederkörper-Drehelement auf. In der äußeren Hülse 16 wird dieses Spiralfederkörper-Drehelement durch zwei diametral gegenüberliegende Ausschnitte 26 z.B., jeweils mit einem einseitigen Fenster 27, gebildet. In der inneren Hülse 20 wird das Spiralfederkörper-Drehelement z.B. von zwei diametral gegenüberliegenden, im Grundzug rechteckigen Ausschnitten 28 gebildet.

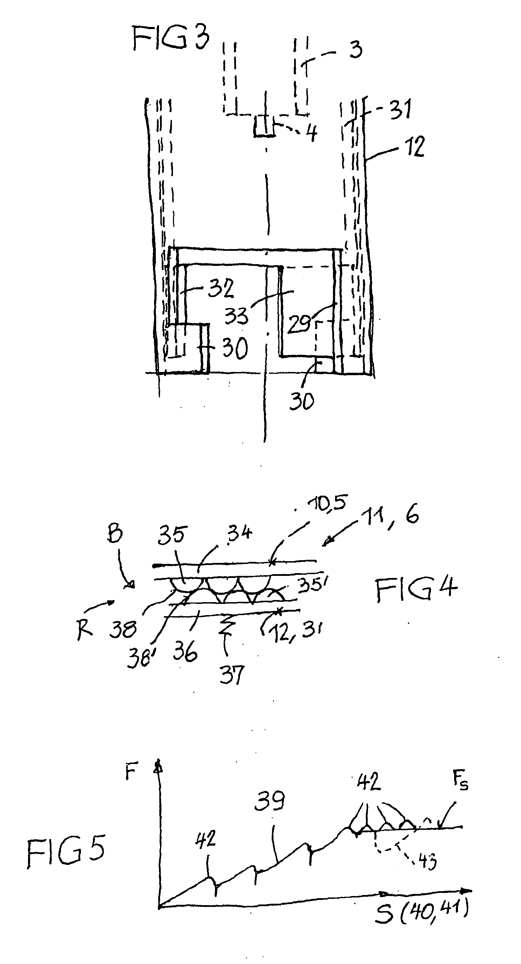

Diese Spiralfederkörper-Drehelemente passen zu Spiralfederkörper-Drehelementrohren 7, 12, die am unteren Ende des Montierinstrumentes W entsprechend Ausführungsformen haben **(****Fig. 3****).**

Im unteren Ende des äußeren Rohres 12 in **Fig. 3** sind zwei diametral gegenüberliegende Fenster 29, z.B. jeweils mit einem Vorsprung 30, geformt, während im unteren Ende des inneren Rohres 31 zwei diametral gegenüberliegende, im Grundzug rechteckige Fenster 32 geformt sind.

Bei der Benutzung des Montierinstrumentes W zum Festsetzen des Marknagels N von **Fig. 2** wird der Vorsprung 4 in die Quernut 13' eingebracht und werden die zwischen den Ausschnitten 32 und 29 stehenden Stege in die Ausschnitte 26, 28 eingebracht und der Vorsprung 30 in das Fenster 27 gedreht. Am Haltegriff 1 wird der Kern des Marknagels N gegen eine Mitdrehung abgestützt, ehe mittels der Drehhandhabe 10 die äußere Hülse 16 so verdreht wird, dass sich der äußere Spiralfederkörper 17 aufweitet und an die Wand des Markkanals anlegt. Zum Aufweiten wird ein begrenztes Drehmoment aufgebracht. Dann wird mit der anderen Drehhandhabe 5 in **Fig. 1** der innere Spiralfederkörper 22 in der anderen Drehrichtung aufgeweitet, bis er den äußeren Spiralfederkörper 17 fixiert. Die Zusammenwirkung zwischen den Spiralfederkörpern ist selbsthemmend, so dass dann das Montierinstrument abgenommen werden kann. Dann wird auf das obere Ende des Marknagels N eine nicht gezeigte Kappe aufgeschraubt.

Damit sich die Spiralfederkörper 17, 22 über die gesamte Windungslänge im Wesentlichen gleichmäßig aufweiten, ist hier jede Drehhandhabe 5, 10 mit einer Drehrastvorrichtung R verbunden, die unter dem aufgebrachten Drehmoment schlagartig variierende Drehkraftimpulse generiert, die in die Spiralfederkörper 17, 22 eingeleitet werden und bewirken, dass die Windungen auch bei lokal unterschiedlichen Reibungs- oder Aufweitwiderständen bis unten hin im Wesentlichen gleichförmig aufgeweitet werden. Jede Drehrastvorrichtung R ist, zweckmäßig, gleichzeitig als Drehmomentbegrenzer B ausgebildet, dessen Grenzdrehmoment wählbar oder vorgegeben ist.

**Fig. 4** verdeutlicht schematisch eine Drehrastvorrichtung R. Ein beispielsweise mit der Drehhandhabe 5 oder 10 verbundenes Glied 34 trägt mehrere Eingriffselemente 35 gleicher Form, die konkav gerundete Eingriffsflächen 38 aufweisen, vorzugsweise Eingriffsflächen, deren Erzeugende ein Kreisbogen oder mehrere Kreisbögen sind. Die Eingriffselemente 35 können Kugeltelle, Zylinderteile oder Konusteile sein.

Mit dem Rohr 12 bzw. 31 ist ein weiteres Glied 36 verbunden, das ebenfalls Eingriffselemente 35' mit konkav gerundeten Eingriffsflächen 38' trägt. Die Eingriffselemente 35, 35' stehen miteinander in einem Formschluss konkav auf konkav, und zwar unter der Wirkung zumindest einer Feder 37. Die Kontaktbereiche sind entweder linienförmig oder punktförmig.

Unter dem aufgebrachten Drehmoment, zumindest kurz vor und bei Erreichen der eingestellten Drehmomentgrenze oder auch schon vorher, gleiten die Eingriffselemente 35, 35' übereinander, wobei sie mehrere schlagartig variierende Drehkraftimpulse 42 **(****Fig. 5****)** erzeugen. Jeder Drehkraftimpuls 42 ist z.B. dadurch gekennzeichnet, dass die Drehkraft am Spiralfederkörperende im Anstieg weitgehend abbricht, gegebenenfalls vollständig abfällt und sich relativ schart wieder auf den vorhergehenden Wert oder höher aufbaut. Der Abfall und der Anstieg pflanzen sich im Spiralfederkörper effizient bis ganz nach unten fort.

**Fig. 5** zeigt im Diagramm ein Beispiel zur Entwicklung der Drehkraft F mittels der Drehrastvorrichtung R über den Drehhub S in der jeweiligen Drehrichtung 40, 41. Eine Kurve 39 verdeutlicht die Zunahme der Drehkraft F mit zunehmendem Drehhub und die durch das Übereinandergleiten der Eingriffselemente 35, 35' von **Fig. 4** generierten, schlagartig variierenden Drehkraftimpulse 42. Auch nach Erreichen der eingestellten Drehkraft Fs, entsprechend dem eingestellten Maximaldrehmoment, ergeben sich aufeinanderfolgende variierende Drehkraftimpulse.

Sollte sich beispielsweise der äußere Spiralfederkörper 17 nur bis zu einem höherliegenden Bereich des Markkanals aufgrund dort lokaler höherer Aufweit- oder Reibwiderstände nicht durchgehend bis zum unteren Ende aufgeweitet haben, dann wird dieser lokale Widerstand durch die schlagartig variierenden Drehkraftimpulse 42 überwunden, wobei beispielsweise gemäß dem gestrichelten Kurventeil 43 das an der Drehhandhabe 10 zu spürende Drehmoment wieder abfällt und sich die Windungen erst dann mit einer weiteren Drehung bis unten ordnungsgemäß aufinreiten und schließlich der Wert Fs der Drehkraft erreicht wird, der dem eingestellten Grenzdrehmoment entspricht.

## Patentansprüche

1. Montierinstrument (W) für einen Marknagel (N) mit zumindest einem durch eine relative Drehbewegung im Markkanal aufweitbaren Spiralfederkörper (17, 22), mit einem am Montierinstrument (W) vorgesehenen Haltegriff (1) für ein Positionierelement (4) und zumindest einer Drehhandhabe (5, 10), die mit einem relativ zum Positionierelement drehbaren Spiralfederkörper-Drehelementrohr (12, 7) in Verbindung steht, **dadurch gekennzeichnet, dass** in der Verbindung zwischen der Drehhandhabe (5, 10) und dem Spiralkörper-Drehelementrohr (12, 7) eine Drehrastvorrichtung (R) angeordnet ist, die während einer Drehung unter einem von der Drehhandhabe auf das Spiralfederkörper-Drehelementrohr aufgebrachten Drehmoment schlagartig variierende Drehkraftimpulse (42) generiert.

2. Montierinstrument gemäß Anspruch 1, **dadurch gekennzeichnet, dass** die Drehrastvorrichtung (R) ein, vorzugsweise einstellbarer, Drehmomentbegrenzer (B) ist.

3. Montierinstrument gemäß Anspruch 1, **dadurch gekennzeichnet, dass** die Drehrastvorrichtung (R) ein federbelastetes Ratschensystem aufweist.

4. Montierinstrument gemäß Anspruch 1, **dadurch gekennzeichnet, dass** die Drehrastvorrichtung (R) unter Federkraft (37), in einen gegenseitigen Formschluss gepresste, zueinander konkave Eingriffselemente (**35**, **35**') enthält.

5. Montierinstrument gemäß Anspruch 4, **dadurch gekennzeichnet, dass** die Eingriffselemente (35, 35') kugelige, zylindrische oder konische Eingriffsflächen (38, 38') aufweisen und miteinander in Punkt- oder Linienkontakt stehen.

6. Montierinstrumentgemäß einem dervorhergehenden Ansprüche, **dadurch gekennzeichnet, dass** für Marknägel (N) mit zwei ineinandergesetzten, gegensinnigen Spiralfederkörpern (17, **22**) und zwei Spiralfederkörper-Drehelementrohren (**12**, 7) zwei getrennte Drehhandhaben (5, 10) vorgesehen sind, und dass zwischen jeder Drehhandhabe und deren Spiralfederkörper-Drehelementrohr eine Drehrastvorrichtung (R) angeordnet ist, die, vorzugsweise, ein Drehmomentbegrenzer (B) ist.

7. Montierinstrument gemäß Anspruch 6, **dadurch gekennzeichnet, dass** jeder Drehmomentbegrenzer (B) auf eine individuelle Drehmomentgrenze eingestellt bzw. einstellbar ist.

8. Montierinstrument nach wenigstens einem der vorhergehenden Ansprüche, **dadurch gekennzeichnet, dass** die Rastvorrichtung (R) ein an der Drehhandhabe (5, 10) angebrachtes Gehäuse (6,11) aufweist, in dem die Eingriffselemente (35, 35') und wenigstens eine Feder (37) untergebracht sind.

9. Montierinstrument gemäß Anspruch 8, **dadurch gekennzeichnet, dass** zwischen dem Gehäuse (6) einer Drehhandhabe (5) und der anderen Drehhandhabe (10) eine Distanzhülse (9) angeordnet ist.

10. Montierinstrument gemäß Anspruch 8, **dadurch gekennzeichnet, dass** das Gehäuse (6, 11) Reinigungsöffnungen (43) aufweist.

11. Montierinstrument gemäß Anspruch 8, **dadurch gekennzeichnet, dass** die Drehhandhabe (5, 10) angrenzend an das Gehäuse (6, 11) randseitige Greifhilfen (44) aufweist.

12. Montierinstrument gemäß Anspruch 8, **dadurch gekennzeichnet, dass** das Gehäuse (6, 11) gleichzeitig als die Drehhandhabe (5, 10) ausgebildet ist.
